Europäisches Patentamt

European Patent Office (11) Publication number: **0 013 783**

Office européen des brevets **B1**

(19)

(12) **EUROPEAN PATENT SPECIFICATION**

(45) Date of publication of patent specification: **04.04.84**

(51) Int. Cl.³: **A 61 K 31/70** //(A61K31/70, 31/60, 31/19)

(21) Application number: **79300053.0**

(22) Date of filing: **12.01.79**

(54) Protection of the gastric mucosal lining from damage by aspirin and related drugs.

(43) Date of publication of application:
06.08.80 Bulletin 80/16

(45) Publication of the grant of the patent:
04.04.84 Bulletin 84/14

(84) Designated Contracting States:
BE CH DE FR GB LU NL SE

(56) References cited:
FR - A - 2 159 194
FR - A - 2 221 128
FR - M - 2 573

"The Salicylates", A Critical Bibliographic
Review, M. Gross and L.A. Greenberg, Hillhouse
Press (1948), p.8 and 9 (Chapter II)
"The Salicylates", A Critical Bibliographic
Review, M.J.H. Smith and P.K. Smith,

The file contains technical information
submitted after the application was filed and not
included in this specification

(73) Proprietor: THE AUSTRALIAN NATIONAL
UNIVERSITY
GBO Box 4
Canberra, 2600 (AU)

(72) Inventor: Rainsford, Kim Drummond, Dr.
5 Binya Court
Kingston Beach Tasmania, 7151 (AU)
Inventor: Whitehouse, Michael Wellesley, Dr.
11 Wolgal Place
Aranda ACT, 2614 (AU)

(74) Representative: Hartley, David et al,
c/o Withers & Rogers 4 Dyer's Buildings Holborn
London, EC1N 2JT (GB)

(56) References cited:
Interscience Publishers (1966), Introduction
The Pharmacological Basis of Therapeutics
(fourth Edition), L.S. Goodman and A. Gillman
(1970), Macmillan Company, p. 314
Principles of Biochemistry (fourth Edition), A.
White, P. Handler and E.L. Smith, McGraw-Hill,
LTD, p. 418 and 419

Courier Press, Leamington Spa, England.

Protection of the gastric mucosal lining from damage by aspirin and related drugs

This invention relates to pharmaceutical formulations for reducing gastrointestinal damage by Acetyl Salicylic acid e.g. aspirin (R.T.M.) and other salicylates, which drug formulations which are safer, in the sense of being less gastrotoxic.

Gastrointestinal damage (ulceration and haemorrhage) is a serious side effect associated with many analgesics and indeed with many such drugs which are readily available without medical prescription in across the counter transactions: Aspirin itself, dispersed in water (it is not very soluble) causes local injury to gastric mucosa in experimental animals—rats, pigs and monkeys as well as in man. Salts of aspirin, e.g. with $Ca^{++}$, are more soluble but still cause gastric irritations and/or ulcerations. These undesirable effects of both soluble and insoluble aspirin, are accentuated by fasting and by stress: consitions that often pertain when the drug is consumed e.g. in the middle of the night to relieve pain and in the early morning to alleviate the stiffness of arthritis. Although bicarbonate had been widely promoted commercially as preventing gastrotoxicity, our investigations showed this to be not so in stressed animals.

French Patent specification 2,159,194 discloses a pharmaceutical composition containing a therapeutic amount of aspirin or a derivative of salicylic acid in association with a lesser amount of sorbitol. A later French specification 2,221,128 discloses the addition of a polyol to aspirin.

Both aim to reduce the ulcerogenic affects of salicylic acid or the derivatives acetyl salicylic acid or sodium salicylate, by the addition of polyols only. However, these proposals are not entirely successful in that only slight reductions in ulcerogenic activity is achieved in the more sensitive assays of gastric ulcerogenic activity (as described in Rainsford 1975, Agents and Actions 5, 533).

Furthermore, recent proposals to use aspirin (or its 3,5-dibromo derivative) to prevent haemoglobin stacking in erythrocytes in "sickles cell disease" and to use aspirin as a prophylactic drug to prevent thrombotic (ischaemic heart) disorders indicate that aspirin will probably remain an essential drug in the therapeutic armoury. Removing the acetyl group of aspirin abolishes both its anti-sickling and its anti-thrombotic effects. However, retention of the acetyl group ensures optimal gastrotoxicity. Therefore further widespread prophylactic use of aspirin may well be determined by any strategy to raise its efficacy:gastrotoxicity ratio.

A prime object of this invention is to provide a formulation which whilst reducing gastrointestinal damage still remains pharmaceutical potency.

According to the present invention, we propose a pharmaceutical formulation for reducing gastrointestinal damage comprising an analgesic selected from the group consisting of the salicylates and their pharmaceutically acceptable salts which tend to cause gastric irritations or ulcerations when used alone as an analgesic, a pharmaceutically acceptable metabolisable carbohydrate a pharmaceutically-acceptable solubilising agent for the analgesic with or without pharmaceutically acceptable excipients, the solubilising agent being selected from alkali metal or alkali earth metal carboxylates and ammonium salts of carboxylic acids and phosphoric acid.

More than one salicylate, metabilisable carbohydrate and solubilising agent may be included in the formulation if desired. Further pharmaceutically acceptable excipients may be added if desired. The formulation may be prepared in solid or liquid form (including dispersed) form and, furthermore, significantly reduces the number and/or the severity of the mucosal lesions or ulcers occurring in the stomach wall that are observed with the same dosage of the salicylate alone.

The preferred analgesic is acetylsalicylic acid and the preferred solubilising agents are sodium acetate and sodium citrate. The preferred metabolisable carbohydrate is D-glucose but others are effective.

Advantages of the present invention will become clearer from a consideration of the following examples.

Example 1

A test formulation (hereinafter called AN-SPIRIN AG) comprising 1 part of aspirin, 3 molar equivalents of sodium acetate and 3 molar equivalents of D-glucose was formulated in liquid form by dissolution in a minimal amount of water. Surprisingly the lesion index observed in the stomach of rats orally adminstered with AN-ASPIRIN AG was only 7 compared with a lesion index of 36 observed in rats treated with a like amount of aspirin (150 mg/kg) dispersed in water. Lesion index is a measure of gastric mucosal damage (see Rainsford, 1975 Agents and Actions 5, 533). The figures in respect of pig-tail monkeys are even more surprising as a monkey orally adminstered with aspirin dispersed in water developed 68 lesions whereas a monkey orally adminstered with a like amount of AN-ASPIRIN AG developed only 6 lesions.

Example 2

In the foregoing Example 1, substitution of sodium citrate for sodium acetate gave analogous results.

Example 3

3,5-Dibromo-o-acetyl salicyclic acid (dibromo-aspirin) at a dose of 150 mg/kg given orally to starved and cold-stressed rats caused gastric haemorrhage within 2 hours, the lesion index was 20.3. Another group of rats, starved and cold-stressed, given orally 150 mg/kg aspirin alone at the same time has a lesion index of 50.8.

Oral administration of 282 mg/kg or dibromo-aspirin to a further group of starved and cold-stressed rats together with 3 molar equivalents of D-glucose and 3 molar equivalents of sodium acetate gave a lesion index of only 3.0.

Example 4

Substitution of an equivalent amount of disodium citrate for the sodium acetate in the foregoing Example 3 gave no lesion at all i.e. the lesion index=0.0.

Tests with formulations containing aspirin and sodium acetate (but no D-glucose), and aspirin and D-glucose (but no sodium acetate) showed much less reduction in the number of lesions and their severity compared with the use of aspirin alone. Consequently the remarkable reduction in the number and severity of gastric mucosal lesions occurring when using AN-ASPIRIN AG is due to the combination of the three ingredients.

As mentioned previously D-glucose is the preferred gastro-protectant. Tests with fructose and sucrose given orally in combination with sodium citrate or sodium acetate and aspirin showed them to be very effective in reducing gastric mucosal damage. Results obtained with galactose showed it was not as effective as the other sugars tested.

As indicated earlier sodium acetate and sodium citrate are preferred solubilising agents. Particularly effective results have been obtained with formulations of aspirin, glucose and tri-sodium citrate in molar proportions of 1:3:3. Replacement of the tri-sodium citrate by disodium citrate gave slightly less effective results.

## Claims

1. A pharmaceutical formulation for reducing gastrointestinal damage comprising an analgesic selected from the group consisting of the salicylates and their pharmaceutically acceptable salts which tend to cause gastric irritations or ulcerations when used alone as an analgesic, a pharmaceutically-acceptable solubilising agent for the analgesic with or without pharmaceutically acceptable excipients, the solubilising agent being selected from alkali metal or alkaline earth metal carboxylates, and ammonium salts of carboxylic acids and phosphoric acid.

2. A pharmaceutical formulation according to claim 1 comprising acetylsalicylic acid, a sodium citrate and D-glucose.

3. A pharmaceutical formulation as claimed in claim 2 wherein the sodium citrate is tri-sodium citrate.

4. A pharmaceutical formulation as claimed in claim 2 wherein the sodium citrate is disodium citrate.

5. A pharmaceutical formulation according to claim 1 comprising acetyl salicylic acid, sodium acetate and D-glucose.

6. A pharmaceutical formulation as claimed in claim 2 wherein the molar proportion of acetylsalicylic acid to sodium citrate to D-glucose is 1:3:3.

7. A pharmaceutical formulation as claimed in claim 1 wherein the analgesic is 3,5-dibromo-o-acetyl salicylic acid.

8. A pharmaceutical formulation according to claim 1 comprising 3,5-dibromo-o-acetyl salicylic acid, sodium acetate and D-glucose.

9. A pharmaceutical formulation as claimed in claim 8 wherein the respective molar proportions of the components are 1:3:3.

## Patentansprüche

1. Pharmazeutische Formulierung zur Verminderung von gastrointestinalen Schädigungen, enthaltend ein Analgetikum, ausgewählt aus der Gruppe bestehend aus Salizylaten und deren pharmazeutisch geeigneten Salzen, welche bein alleiniger Verwendung als Analgetikum zur Verursachung von gastrischen Reizungen oder Magengeschwüren neigen, ein pharmazeutisch geeignetes, dem Stoffwechsel zugängliches Kohlenhydrat, ein pharmazeutisch geeignetes Lösungsmittel für das Analgetikum mit oder ohne pharmazeutisch geeignete Arzneimittelträger, wobei das Lösungsmittel aus Alkalimetall- oder Erdalkali-Carboxylaten ausgewählt ist, sowie Ammoniumsalze von Carbonsäuren und der Phosphorsäure.

2. Pharmazeutische Formulierung nach Anspruch 1, dadurch gekennzeichnet, daß sie Acetylsalicylsäure, ein Natriumcitrat und D-Glukose enthält.

3. Pharmazeutische Formulierung nach Anspruch 2, dadurch gekennzeichnet, daß das Natriumcitrat Trinatriumcitrat ist.

4. Pharmazeutische Formulierung nach Anspruch 2, dadurch gekennzeichnet, daß das Natriumcitrat Dinatriumcitrat ist.

5. Pharmazeutische Formulierung nach Anspruch 1, dadurch gekennzeichnet, daß sie Acetylsalicylsäure, Natriumacetat und D-Glukose enthält.

6. Pharmazeutische Formulierung nach Anspruch 2, dadurch gekennzeichnet, daß das Molverhältnis von Acetylsalicylsäure zu Natriumcitrat zu D-Glukose 1:3:3 ist.

7. Pharmazeutische Formulierung nach Anspruch 1, dadurch gekennzeichnet, daß das Analgetikum 3,5-Dibromo-acetylsalicylsäure ist.

8. Pharmazeutische Formulierung nach Anspruch 1, dadurch gekennzeichnet, daß sie 3,5-Dibrom-3-acetylsalicylsäure, Natriumacetat und D-Glukose enthält.

9. Pharmazeutische Formulierung nach Anspruch 8, dadurch gekennzeichnet, daß das Molverhältnis der Komponenten 1:3:3 ist.

**Revendications**

1. Une formulation pharmaceutique destinée à réduire les dommages gastrointestinaux comprenent un analgésique choisi parmi le groupe comprenant les salicylates et leurs sels pharmaceutiquement acceptables qui tendent à provoquer des ulcérations ou irritations gastriques lorsqu'ils sont utilisés seuls comme analgésiques, un hydrate de carbone métabolisable pharmaceutiquement acceptable, un agent de solubilisation pharmaceutiquement acceptable pour l'analgesique avec ou sans excipients pharmaceutiquement acceptables, l'agent solubilisant étant choisi parmi les carboxylates de métaux alcalins ou alcalino-terreux et les sels d'ammonium des acides carboxyliques et de l'acide phosphorique.

2. Une formulation pharmaceutique selon la revendication 1, comprenent l'acide acétylesalicylique, un citrate de sodium et du D-glucose.

3. Une formulation pharmaceutique selon la revendication 2, dans laquelle le citrate de sodium est le citrate tri-sodique.

4. Une formulation pharmaceutique selon la revendication 2, dans laquelle le citrate de sodium est le citrate di-sodique.

5. Une formulation pharmaceutique selon la revendication 1, comprenant de l'acide acétylesalicylique, de l'acétate de sodium et du D-glucose.

6. Une formulation pharmaceutique selon la revendication 2, dans laquelle les proportions molaires de l'acide acétylsalicylique du citrate de sodium et du D-glucose sont dans les rapports 1/3/3.

7. Une formulation pharmaceutique selon la revendication 1, dans laquelle l'analgésique est l'acide 3,5-dibromo-o-acétylsalicylique.

8. Une formulation pharmaceutique selon la revendication 1, comprenant de l'acide 3,5-dibromo-o-acétylesalicyclique, de l'acétate de sodium et du D-glucose.

9. Une formulation pharmaceutique selon la revendication 8, dans laquelle les proportions respectives molaires des composants sont de 1/3/3.